# FASCICULE DE BREVET EUROPEEN

(11) **EP 0 753 518 B1**
(45) Date de publication et mention de la délivrance du brevet: **20.09.2000**
(21) Numéro de dépôt: 96401252.0
(22) Date de dépôt: 11.06.1996
(51) Int. Cl.: C07D 487/06, A23K 1/16

(54) **Procédés pour la préparation d'une forme cristallisée particulière de chlorohydrate de zilpatérol, et les produits intermédiaires mis en oeuvre**
Verfahren zur Herstellung einer Kristallinen Sonderform von Zilpaterolhydrochlorid, und benutze Zwischenprodukte
Processes for the preparation of a specific crystalline form of zilpaterol hydrochloride, and intermediates used

(30) Priorité: 13.06.1995 FR 9506966
(43) Date de publication de la demande: 15.01.1997
(73) Titulaire: Hoechst Roussel Vet S.A., 93230 Romainville (FR)
(72) Inventeur: Chevremont, Yves, 77230 Villeneuve sous Dammartin (FR); Godard, Jean-Yves, 93340 Le Raincy (FR)
(74) Mandataire: Van Gent, Marieke

(56) Documents cités:
- EP-A- 0 107 569
- FR-A- 2 608 046
- DATABASE REGISTRY CHEMICAL ABSTRACTS SERVICE, COLUMBUS, OHIO, US (STN), XP002011858 & CHEMICAL ABSTRACTS, vol. 114, no. 25, 24 Juin 1991 Columbus, Ohio, US; abstract no. 246296, & FR-A-2 647 310 (ROUSSEL-UCLAF SA)

## Description

La présente invention concerne le chlorhydrate de zilpatérol sous une forme cristallisée particulière, son procédé de préparation et les produits intermédiaires mis en oeuvre.

L'invention a pour objet un procédé de préparation du chlorhydrate de zilpatérol anhydre sous forme cristallisée, caractérisé en ce qu'il comprend moins de 5 % de cristaux d'une taille inférieure à 15 microns, le reste des cristaux ayant une taille inférieure à 250 microns.

Le chlorhydrate de zilpatérol est un produit connu pour ses propriétés biologiques, qui peut être utilisé chez les animaux d'élevage comme il est indiqué par exemple dans le brevet français 2608046.

Le chlorhydrate de zilpatérol permet d'améliorer la prise de poids et la qualité de la viande chez les animaux d'élevage, par exemple les bovins et les porcins.

Le chlorhydrate de zilpatérol peut être additionné à la nourriture pour animaux.

On peut de façon intéressante préparer un prémix qui est ensuite incorporé à la nourriture.

Un procédé de préparation de prémix intéressant consiste à coller le chlorhydrate de zilpatérol sur des rafles de mais, en utilisant par exemple le procédé décrit dans la demande de brevet européen 197188.

Les particules de principe actif doivent être de taille inférieure à celle du support.

Pour un collage sur des rafles de mais qui ont une taille comprise entre 300 et 800 microns, il est souhaitable que la totalité des particules de principe actif ait une taille inférieure à 300 microns et que la majorité ait une taille comprise entre 50 et 200 microns. Il est aussi souhaitable que les particules de principe actif ne soient pas trop fines si l'on veut éviter les problèmes de poussière.

Lorsque les particules de principe actif sont trop fines, et se présentent sous forme de poussière, il y a un problème d'hygiène pour les utilisateurs qui peuvent être gênés, voire intoxiqués, par exemple lorsque le principe actif pénètre dans les alvéoles pulmonaires. Il y a de plus des problèmes graves pour l'environnement.

Il était donc souhaitable d'obtenir le chlorhydrate de zilpatérol sous une forme granulométrique appropriée répondant aux normes exposées ci-dessus.

L'invention a pour objet un procédé de préparation, caractérisé en ce que l'on
1 - prépare une solution sursaturée de chlorhydrate de zilpatérol dans l'eau, ou dans un mélange eau/alcool éthylique à une température supérieure à 50°C,
2 - refroidit la solution ainsi obtenue et obtient la cristallisation du monohydrate,
3 - abaisse la température au-dessous de 20°C et obtient la cristallisation du trihydrate,
4 - sèche et obtient ainsi le produit microcristallisé sous sa forme anhydre.

L'invention a également pour objet une variante du procédé, caractérisée en ce que l'on dissout dans l'eau du chlorhydrate de zilpatérol anhydre au-dessous de 30°C, obtient une solution aqueuse saturée en chlorhydrate de zilpatérol qui recristallise spontanément sous forme de trihydrate.

L'invention a en outre une variante du procédé précédent, caractérisé en ce que l'on dissout le chlorhydrate de zilpatérol dans un volume minimum d'eau à chaud, entre 60 et 100°C, verse cette solution sur une solution saturée du chlorhydrate de zilpatérol dans un mélange eau et alcool, agitée à une température inférieure à 20°C et préalablement ensemencée avec des germes de trihydrate et obtient ainsi le trihydrate que l'on sèche pour obtenir le produit recherché.

La partie expérimentale exposée ci-après indique les conditions préférentielles du procédé de l'invention.

Le monohydrate du chlorhydrate de zilpatérol est un produit nouveau et est en lui-même un objet de la présente invention.

Le trihydrate du chlorhydrate de zilpatérol est également un produit nouveau et est ainsi en lui-même un objet de la présente invention.

Les exemples suivants illustrent l'invention sans toutefois la limiter.

### Préparation 1

On introduit dans un réacteur en une fois 400 g de chlorhydrate de zilpatérol, 480 ml d'eau et 240 ml d'éthanol. On chauffe la suspension à 70°C et obtient après 15 mn la dissolution totale. La solution est ensuite refroidie à 45°C, maintenue ainsi pendant 30 minutes à partir du début de la cristallisation de la forme monohydrate. On peut vérifier par un simple examen au microscope optique la cristallisation en aiguilles, caractéristique du monohydrate. La suspension est ensuite refroidie à 10°C régulièrement en 35 minutes. La suspension épaisse ainsi obtenue est ensemencée avec 0,2 g de forme trihydrate micronisée (H₂O = 13 %, 97 % de particules < 10 µ).

La suspension est agitée à 10°C jusqu'à la fin de la transformation de la forme monohydrate en forme trihydrate. On peut vérifier à l'aide d'un microscope optique la cristallisation granulaire et l'absence de cristaux en aiguilles. La durée de la transformation varie de 5 à 17 heures. La suspension est alors chauffée à 30°C pendant 16 heures puis refroidie à 0°C régulièrement en 2 heures. Après un contact de 2 heures à 0°C, le produit est essoré et lavé avec 100 ml d'eau à 33 % d'éthanol. Les cristaux sont séchés pendant 16 heures à 20°C sous pression réduite (15 torr) puis à 60°C pendant 24 heures toujours sous pression réduite en présence de potasse en pastilles. Le produit sec est ensuite passé sur un tamis de 0,6 mm. On obtient ainsi 358 g de chlorhydrate de zilpatérol microcristallisé (eau = 0,4 %, éthanol = 0,05 %, taille des particules : 3 % < 15 µ, 93 % < 200 µ, 100 % < 245 µ).

### Préparation 2

On introduit dans un réacteur 200 ml d'eau que l'on chauffe à 30°C. On ajoute 160 g de chlorhydrate de zilpatérol anhydre en branche, rapidement. Il y a dissolution pratiquement complète. Après environ 10 mn, la cristallisation se développe spontanément. L'observation des cristaux au microscope optique montre qu'il s'agit bien de la forme trihydratée (cristaux de forme granulaire). Après 1 heure trente de contact à 30°C, on refroidit la suspension à 0°C régulièrement en 1 heure puis agite pendant 2 heures à 0°C. On essore le produit puis les liqueurs mères sont déplacées par un lavage avec 40 ml d'eau. Le produit est séché dans les conditions de la préparation 1. On obtient ainsi 144 g de chlorhydrate de zilpatérol microcristallisé que l'on tamise sur un jeu de tamis en acier inoxydable placé sur un vibreur tridimensionnel. On récupère 137 g au refus de tamis 50 µ (eau = 0,65 %, taille des particules : 1 % < 15 µ, 92 % < 200 µ, 99 % < 250 µ).

### Préparation 3

Dans un réacteur on dissout 30 g de chlorhydrate de zilpatérol dans 300 ml d'éthanol à 50 % d'eau. On refroidit la solution à + 15°C, ensemence avec 4 g de trihydrate broyé (H₂O = 11,8 %, taille des particules : 60 % < 50 µ). On ajoute alors régulièrement en 3 heures sous agitation à + 15°C, une solution de 210 g de chlorhydrate de zilpatérol dans 300 ml d'éthanol à 50 % d'eau maintenue à 85°C environ. Puis la suspension est chauffée à + 27°C et agitée pendant 20 heures à 27°C. On refroidit ensuite cette suspension à 0°C régulièrement en 1 heure 30 et agite à 0°C pendant 2 heures. La suspension est alors essorée et les liqueurs mères déplacées à 0°C avec 80 ml d'éthanol à 50 % d'eau. Les cristaux isolés sont séchés sous pression réduite dans les conditions de la préparation 1. On récupère 215 g de chlorhydrate de zilpatérol microcristallisé (eau = 1,3 %, taille des particules : 4 % < 15 µ, 90 % < 200 µ,
99 % < 250 µ).

### Caractéristiques physico-chimiques

### Spectre infrarouge

**Tab. 1 :**

| Les bandes caractéristiques de chaque polymorphe sont les suivantes : | | | |
|---|---|---|---|
| Forme | Anhydre | Monohydrate | Trihydrate |
| | 3280 | 3380 | 3470 |
| | 3180 | 3230 | 3350 |
| Nombre d'onde | 1705 | 3180 | 3100 |
| de la bande | 1600 | 1690 | 1670 |
| caractéristique | 785 | 1610 | 1620 |
| (cm⁻¹) | 745 | 795 | 785 |
| | | 785 | 750 |
| | | 750 | |
| | | 735 | |

### Analyse calorimétrique différentielle

Chaque polymorphe a été caractérisé également par son comportement en analyse calorimétrique différentielle (ADC).

**Tab. 2 :**

| Caractéristiques DSC de chaque polymorphe : | | |
|---|---|---|
| Caractéristiques ADC | | |
| Forme anhydre | Monohydrate | Trihydrate |
| endotherme à 219°C | endothermes à 170°C | endothermes à 64°C |
| | 219°C | 103°C |
| | | 232°C |

### Diagramme de diffraction des poudres aux rayons X

Les diagrammes ont été obtenus avec la radiation Ka du cuivre à une longueur d'onde λ = 1,54 A. Dans le tableau ci-dessous, D et I/I₁, représentent respectivement les espacements inter-réticulaires et les intensités relatives.

**Tab. 3 :**

| Caractéristiques RX de chaque polymorphe : | | | | | |
|---|---|---|---|---|---|
| Forme anhydre | | Monohydrate | | Trihydrate | |
| D | I/I₁ | D | I/I₁ | D | I/I₁ |
| 10,99 | 0,92 | 42,44 | 0,20 | 9,18 | 0,669 |
| 8,59 | 0,20 | 12,59 | 0,19 | 7,92 | 0,14 |
| 7,79 | 0,51 | 7,73 | 1,00 | 6,86 | 0,20 |
| 6,09 | 0,55 | 7,00 | 0,19 | 6,10 | 0,29 |
| 5,93 | 0,48 | 6,63 | 0,13 | 5,32 | 0,13 |
| 5,82 | 0,34 | 6,09 | 0,15 | 5,04 | 0,18 |
| 5,29 | 0,24 | 5,93 | 0,16 | 4,79 | 0,17 |
| 4,30 | 0,37 | 5,75 | 0,13 | 4,73 | 0,23 |
| 4,20 | 0,28 | 5,63 | 0,13 | 4,59 | 0,18 |
| 3,98 | 0,33 | 5,51 | 0,13 | 4,27 | 0,16 |
| 3,90 | 0,38 | 4,91 | 0,18 | 3,98 | 0,22 |
| 3,72 | 0,84 | 4,52 | 0,17 | 3,83 | 1,00 |
| 3,59 | 1,00 | 4,27 | 0,21 | 3,72 | 0,30 |
| 3,45 | 0,47 | 4,21 | 0,21 | 3,58 | 0,14 |
| 2,88 | 0,31 | 3,90 | 0,22 | 3,49 | 0,74 |
| 2,71 | 0,18 | 3,82 | 0,27 | 3,45 | 0,26 |
| | | 3,75 | 0,27 | 3,34 | 0,66 |
| | | 3,71 | 0,33 | 3,23 | 0,95 |
| | | 3,61 | 0,19 | 3,08 | 0,14 |
| | | 3,51 | 0,36 | 3,06 | 0,14 |
| | | 3,46 | 0,34 | 2,99 | 0,13 |
| | | 3,35 | 0,31 | 2,96 | 0,13 |
| | | 3,32 | 0,20 | 2,82 | 0,13 |
| | | 3,28 | 0,16 | 2,69 | 0,10 |
| | | 3,21 | 0,24 | 2,63 | 0,15 |
| | | 3,14 | 0,23 | 2,60 | 0,17 |
| | | 3,12 | 0,16 | 2,50 | 0,12 |
| | | 3,05 | 0,12 | 2,43 | 0,12 |
| | | 2,90 | 0,13 | | |
| | | 2,80 | 0,14 | | |
| | | 2,68 | 0,11 | | |
| | | 2,64 | 0,12 | | |
| | | 2,60 | 0,13 | | |
| | | 2,50 | 0,14 | | |
| | | 2,45 | 0,10 | | |

### Détermination de la structure du trihydrate par diffraction RX

Il est très difficile d'obtenir des cristaux stabilisés à 3 molécules d'eau car déjà dans l'atmosphère du laboratoire le trihydrate perd naturellement son eau d'hydratation pour évoluer lentement vers la forme anhydre. Seule la préparation de monocristaux de la forme trihydratée a permis de confirmer grâce aux rayons X, la présence de 3 molécules d'eau par mole de chlorhydrate de zilpatérol (CZ). Le système cristallin, les paramètres de maille, le groupe d'espace et les coordonnées atomiques ont pu être ainsi déterminés.

| **Système cristallin** | triclinique |
|---|---|
| a (A) | 8,080 |
| b (A) | 10,268 |
| c (A) | 11,884 |
| alpha (degré) | 113,51 |
| beta (degré) | 90,65 |
| gamma (degré) | 102,34 |
| Groupe d'espace | P1⁻ |
| Z | 2 |
| R | 6,6 |

| **Coordonnées atomiques** | | | | |
|---|---|---|---|---|
| Atom | x/a | y/b | z/c | U(iso) |
| N(3) | 0.4065(4) | 0.2628(4) | -0.0120(3) | 0.0193 |
| C(7) | 0.2882(5) | 0.4401(5) | 0.3820(4) | 0.0201 |
| C(17) | 0.3973(5) | 0.5156(5) | 0.3268(4) | 0.0215 |
| C(15) | 0.3405(5) | 0.3057(5) | 0.1118(4) | 0.0211 |
| C(9) | 0.4474(5) | 0.4538(4) | 0.1967(4) | 0.0206 |
| C(8) | 0.2511(5) | 0.5136(5) | 0.5029(4) | 0.0231 |
| N(4) | 0.2008(5) | 0.2939(4) | 0.3449(3) | 0.0247 |
| O(2) | 0.4486(4) | 0.5546(3) | 0.1403(3) | 0.0235 |
| C(11) | 0.1122(6) | 0.2777(5) | 0.4390(4) | 0.0280 |
| C(14) | 0.4657(6) | 0.6642(5) | 0.3992(4) | 0.0268 |
| C(18) | 0.2821(6) | 0.1423(5) | -0.1169(4) | 0.0276 |
| C(12) | 0.3426(7) | 0.1890(5) | 0.1599(4) | 0.0273 |
| C(21) | 0.3124(6) | 0.6610(5) | 0.5710(4) | 0.0297 |
| N(10) | 0.1406(5) | 0.4126(4) | 0.5330(4) | 0.0289 |
| C(13) | 0.4260(6) | 0.7355(5) | 0.5177(4) | 0.0305 |
| C(23) | 0.1943(7) | 0.1669(5) | 0.2302(5) | 0.0319 |
| C(19) | 0.3766(7) | 0.0613(6) | -0.2193(5) | 0.0372 |
| O(5) | 0.0251(5) | 0.1608(4) | 0.4340(3) | 0.0375 |
| C(20) | 0.1591(7) | 0.2100(7) | -0.1595(6) | 0.0431 |
| CL(1) | 0.2366(2) | 0.8016(1) | 0.9663(1) | 0.0376 |
| O(6) | 0.2083(4) | 0.7071(4) | 1.1889(4) | 0.0390 |
| O(16) | -0.1916(5) | 0.5875(4) | 0.0697(4) | 0.0497 |
| O(22) | 0.0405(7) | 0.5001(5) | 0.7688(4) | 0.0598 |

### Conclusions :

La granulométrie souhaitée a été obtenue.

30 kg de prémix à 3 p. cent masse/masse ont été préparés selon le procédé du brevet européen N° 0197188 à partir du produit préparé selon le procédé 1.

Le premix obtenu a satisfait au test de Heubach, qui est un test bien connu, comme il est indiqué par D. Pickard dans "Feed Compounder" 1992, p. 18 et suivantes.

### Test Heubach :

Générer les poussières au moyen de l'appareil Heubach modifié dans les conditions suivantes :
Taille de l'échantillon : 50 g de premix
Vitesse de rotation du tambour : 30 t/m
Durée de la rotation : 5 mn
Débit d'entrée d'air : 4 L/mn
Porosité du filtre : 0,45 µm.

Le produit retenu sur le filtre est ensuite extrait par sonication dans du méthanol et dosé par HPLC.

### Résultat du test : < 0,1 µg de Zilpatérol par filtre.

Le test de Heubach montre que les cristaux obtenus sont parfaitement adaptés pour leur appliquer le procédé de préparation du premix décrit dans la demande de brevet européen 197188.

## Revendications

1. Procédé de préparation du chlorhydrate de zilpatérol anhydre sous forme cristallisée comprenant moins de 5 % de cristaux d'une taille inférieure à 15 microns, le reste des cristaux ayant une taille inférieure à 250 microns caractérisé en ce que l'on
1 - prépare une solution sursaturée de chlorhydrate de zilpatérol dans l'eau, ou dans un mélange eau-alcool éthylique à une température supérieure à 50°C,
2 - refroidit la solution ainsi obtenue et obtient la cristallisation du monohydrate,
3 - abaisse la température au-dessous de 20°C et obtient la cristallisation du trihydrate,
4 - sèche et obtient ainsi le produit recherché.

2. Variante du procédé de la revendication 1, caractérisée en ce que l'on dissout dans l'eau du chlorhydrate de zilpatérol anhydre au-dessous de 30°C, obtient une solution aqueuse saturée en chlorhydrate de zilpatérol qui recristallise spontanément sous forme de trihydrate que l'on sèche pour obtenir le produit microcristallisé sous sa forme anhydre.

3. Variante du procédé selon la revendication 1, caractérisée en ce que l'on dissout le chlorhydrate de zilpatérol dans un volume minimum d'eau à chaud, entre 60 et 100°C, verse cette solution sur une solution saturée du chlorhydrate de zilpatérol dans un mélange eau et alcool, agitée à une température inférieure à 20°C et préalablement ensemencée avec des germes de trihydrate, obtient ainsi le trihydrate que l'on sèche pour obtenir le produit recherché.

4. A titre de produit chimique nouveau, le monohydrate du chlorhydrate de zilpatérol.

5. A titre de produit chimique nouveau, le trihydrate du chlorhydrate de zilpatérol.

## Patentansprüche

1. Verfahren zur Herstellung von wasserfreiem Zilpaterolchlorhydrat in kristallisierter Form, umfassend weniger als 5% Kristalle mit einer Größe kleiner als 15 Mikrometer, wobei der Rest der Kristalle eine Größe von kleiner 250 Mikrometern besitzt, dadurch **gekennzeichnet**, daß man
1 - eine mit Zilpaterolchlorhydrat übersättigte Lösung in Wasser oder in einem Gemisch aus Wasser-Ethylalkohol bei einer Temperatur über 50°C herstellt,
2 - die so erhaltene Lösung abkühlt und die Kristallisation des Monohydrats erhält,
3 - die Temperatur unter 20°C senkt und die Kristallisation des Trihydrats erhält,
4 - trocknet und so das gewünschte Produkt erhält.

2. Variante des Verfahrens nach Anspruch 1, dadurch **gekennzeichnet**, daß man wasserfreies Zilpaterolchlorhydrat unterhalb von 30°C in Wasser auflöst, eine an Zilpaterolchlorhydrat gesättigte wässrige Lösung erhält, die spontan in Form des Trihydrats kristallisiert, das getrocknet wird, um das mikrokristallisierte Produkt in seiner wasserfreien Form zu erhalten.

3. Variante des Verfahrens nach Anspruch 1, dadurch **gekennzeichnet**, daß man das Zilpaterolchlorhydrat in einem minimalen Volumen heißen Wassers zwischen 60 und 100°C auflöst, diese Lösung auf eine an Zilpaterolchlorhydrat gesättigte Lösung in einem Gemisch aus Wasser und Alkohol gießt, die bei einer Temperatur unter 20°C bewegt wird und zuvor mit Trihydratkeimen angeimpft worden war, so das Trihydrat erhält, das getrocknet wird, um das gewünschte Produkt zu erhalten.

4. Zilpaterolchlorhydratmonohydrat als neues chemisches Produkt.

5. Zilpaterolchlorhydrattrihydrat als neues chemisches Produkt.

## Claims

1. Process for the preparation of anhydrous zilpaterol hydrochloride in crystallized form comprising less than 5 % of crystals of a size less than 15 microns, the remainder of the crystals having a size less than 250 microns characterized in that
1 - a supersaturated solution of zilpaterol hydrochloride is prepared in water, or in a water-ethyl alcohol mixture at a temperature greater than 50°C,
2 - the solution thus obtained is cooled down and crystallization of the monohydrate is obtained,
3 - the temperature is lowered to below 20°C and crystallization of the trihydrate is obtained,
4 - drying is carried out and in this way the sought product is obtained.

2. Variant of the process of claim 1, characterized in that the anhydrous zilpaterol hydrochloride is dissolved in water at below 30°C, a saturated aqueous solution of zilpaterol hydrochloride is obtained which recrystallizes spontaneously in the form of the trihydrate which is dried in order to obtain the microcristallized product in its anhydrous form.

3. Variant of the process according to claim 1, characterized in that the zilpaterol hydrochloride is dissolved in a minimum volume of hot water, between 60 and 100°C, this solution poured into a saturated solution of zilpaterol hydrochloride in a water and alcohol mixture, agitation is carried out at a temperature below 20°C and seeded beforehand with trihydrate crystals, in this way the trihydrate is obtained which is dried in order to obtain the sought product.

4. The monohydrate of zilpaterol hydrochloride as a novel chemical product.

5. The trihydrate of zilpaterol hydrochloride as a novel chemical product.
